(19) Europäisches Patentamt
European Patent Office
Office européen des brevets

(11) Veröffentlichungsnummer: **0 294 538 B1**

# (12) EUROPÄISCHE PATENTSCHRIFT

(45) Veröffentlichungstag der Patentschrift: **29.07.92**

(51) Int. Cl.$^5$: **A61K 31/71**

(21) Anmeldenummer: **88101018.5**

(22) Anmeldetag: **25.01.88**

## (54) Verwendung von Nigerizin zur Herstellung eines Mittels zur Behandlung von Virus-Erkrankungen.

(30) Priorität: **11.06.87 BG 80140/87**

(43) Veröffentlichungstag der Anmeldung:
**14.12.88 Patentblatt 88/50**

(45) Bekanntmachung des Hinweises auf die Patenterteilung:
**29.07.92 Patentblatt 92/31**

(84) Benannte Vertragsstaaten:
**BE CH DE ES FR GB IT LI NL SE**

(56) Entgegenhaltungen:

**PHARMACOLOG. THERAP., Band 23, Nr. 1, 1983, Seiten 109-145; L. CARRASCO et al.: "Permeabilization of cells during animal virus infection"**

**THE JOURNAL OF BIOLOGICAL CHEMISTRY, Band 252, Nr. 6, 26. März 1977, Seiten 1990-1997, US; J.E. LEVER: "Active amino acid transport in plasma membrane vesicles from Simian virus 40-transformed mouse fibroblasts"**

(73) Patentinhaber: **S O "PHARMACHIM"**
**Iliensko Chaussée 16**
**Sofia(BG)**

(72) Erfinder: **Dundarov, Stefan Georgiev**
**Dunav-Str. 58-2**
**BG-1202 Sofia(BG)**
Erfinder: **Andonov, Peter Stoynev**
**Boul. P. Slaveykov 18-2**
**Sofia(BG)**
Erfinder: **Todorov, Tzoni Penev**
**Beli-mel-Str. 4**
**BG-1156 Sofia(BG)**
Erfinder: **Tishkov, Sava Haralampiev**
**T. Strashimirov-Str. 7**
**BG-1504 Sofia(BG)**
Erfinder: **Minkov, Evgeni Hristov**
**Komplex Mladost-2, Bl. 227-A**
**Sofia(BG)**
Erfinder: **Boyadjieva, Nadka Ivanova**
**Ouartal "Istok" P. Degeiter Str. Bl. 1, App. 40**
**Sofia(BG)**
Erfinder: **Marinov, Alexander Nikolov**
**Boul. G. Traykov Bl. 15-22**
**Sofia(BG)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

J. CELL. BIOL., Band 97, Nr. 3, 1983, Seiten 659-668; V. FIRELLI et al.: "Modulation of glycosylation and transport of viral membrane glycoproteins by a sodium ionophore"

Erfinder: **Vulova, Nedka Lyubenova**
**Block "Belmeken" App. 15**
**Razgrad(BG)**
Erfinder: **Alkusheva, Margarita Yordanova**
**Simeon-Str. 1 App. 19**
**Razgrad(BG)**
Erfinder: **Kovatchev, Ivan Todorov**
**Block "Antibiotik" A**
**Razgrad(BG)**
Erfinder: **Ivanov, Zivko Hristov**
**Zavodski Shilishta Bl. A, App. 9**
**Razgrad(BG)**

(74) Vertreter: **von Füner, Alexander, Dr. et al**
**Patentanwälte v. Füner, Ebbinghaus, Finck**
**Mariahilfplatz 2 & 3**
**W-8000 München 90(DE)**

**Beschreibung**

Die Erfindung betrifft die Verwendung eines Antibiotikums zur Herstellung eines Mittels zur Behandlung von Virus-Erkrankungen, verursacht durch DNS enthaltende Viren, wie Herpes-Viren, Adeno-Viren und Pocken-Viren.

Es ist bekannt, zur Behandlung von durch Herpes-, Adeno- und andere DNS enthaltende Viren verursachten Erkrankungen Idoxyridin-(2'-desoxy-5-iodo-uridin) (GB-A-1 024 156), das ein synthetisches, Halogen enthaltendes Purinanalog ist, und Acyclovir (DE-A-2 539 963), das ein Purin-Nukleosidanalog (2-Amino-1,9-dihydro-9-[2-hydroxyethoxy]-methyl-6H-purin-6-on) ist, zu verwenden. Diese Wirkstoffe werden bei Augenerkrankungen in Form von Augentropfen oder Salben, d.h. als 0,1- bis 0,5%ige Lösung oder 1%ige Salbe verwendet. Sie werden auch örtlich in Form von 3%iger Salbe oder 5%iger Creme, 4 bis 6 mal täglich, während 5 bis 10 Tagen angewendet. Acyclovir wird auch systemisch in Tabletten mit je 200 mg Wirkstoff verwendet.

Es ist auch die Anwendung von Gamma-Globulin und die Autohämotherapie zur Behandlung von durch DNS enthaltende Viren verursachten Erkrankungen bekannt sowie auch physiko-chemische Mittel, wie Abrasio (Korrektion von durch Viren geschädigten Geweben), Touchieren mit Jodtinktur (oder beide Verfahren kombiniert) und die Kryotherapie durch Verbrennung bei niedriger Temperatur.

Die Nachteile dieser bekannten Antivirenmittel sind die Entwicklung von resistenten Mutanten, einschließlich auch bei Kombination mehrerer Wirkstoffe, die schwere Löslichkeit, was ihre Anwendung beschränkt, die Entwicklung von Toxizität und anderer Nebenerscheinungen, wie Auftreten von Schmerzen im Auge, Trübewerden der Hornhaut, Auftreten von Konjunktiviten, Blephariten und andere Ulzerose-Beschädigungen der Hornhaut und nicht zuletzt, daß die physiko-chemischen Mittel in der Praxis unwirksam sind.

Der Erfindung liegt die Aufgabe zugrunde, ein wirksames Mittel zur Behandlung von Virus-Erkrankungen, die durch DNS enthaltende Viren verursacht wurden, zu entwickeln, das keine Resistenz und Nebenerscheinungen hervorruft, eine gute Löslichkeit und eine verhältnismäßig niedrige Toxizität aufweist.

Diese Aufgabe wird durch die Verwendung eines Antibiotikums, produziert durch Streptomyces hygroscopicus, als Wirkstoff mit folgender Formel

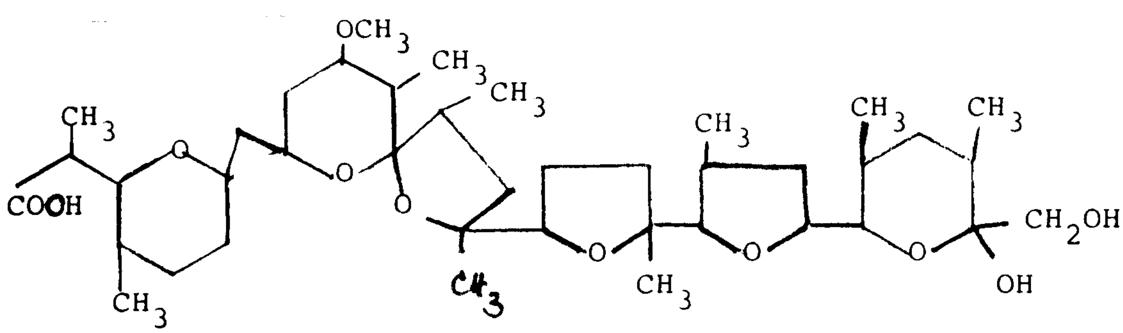

zur Herstellung eines Mittels zur Behandlung von Viruserkrankungen, die durch DNS enthaltende Viren verursacht werden, gelöst.

Dieses Antibiotikum, Nigerizin, das erfindungsgemäß den Wirkstoff das Arzneimittels darstellt, ist in struktureller Hinsicht ähnlich dem Monensin, das als antimikrobielles Antibiotikum mit schwacher antiviraler Wirkung bekannt ist. Nigerizin ist seinerseits als Kokzidiostatikum, bekannt (US-A-3 555 150).

Das erfindungsgemäße Mittel kann in seinen verschiedenen pharmazeutischen Formen von 0,001 bis 3,0 % Nigerizin enthalten. Es wird zur Behandlung von solchen Erkrankungen, die durch DNS enthaltende Viren, wie Labial-, Fazial-, Genitalund Glutealherpes, Herpes Zoster, Herpes- und Adenovirus-Keratitis und Kerato-Konkunktivitis, Herpes-stomatitis, verursacht werden, empfohlen. Die Behandlung wird insbesondere als örtliche Behandlung durchgeführt. Falls das erfindungsgemäß erhaltene Mittel in Form von Augentropfen vorliegt, wird der Patient mit 1 Tropfen pro Stunde behandelt, während, falls das Mittel als Salbe verwendet wird, das Mittel 4 bis 6 mal täglich eingerieben wird. Die Behandlung dauert 3 bis 10 Tage.

Die Vorteile des erfindungsgemäßen Mittels bestehen in seiner höheren antiviralen Wirkung bei 10 bis 1000 mal niedrigerer Molarkonzentration, der hohen Wirksamkeit der Behandlung, dem Wegfall der Entwicklung von resistenten Mutanten, dem Nichtvorhandensein von Nebenerscheinungen, den breiten Grenzen zwischen toxischen und wirksamen Konzentrationen, der guten Löslichkeit des Antibiotikums und der Stabilität des Arzneimittels.

Beispiel 1

Augentropfen: Bestandteile in g pro 10 ml:
Nigerizin - 0,001 bis 0,0001; Natriumchlorid - 0,046; Kalium-Monophosphat - 0,0116; Dinatriumphosphat - 0,1034; Hydroxyethylcellulose - 0,1; Timerosal - 0,0002; Ethanol 95% - 0,005; Twin-80 - 0,005; Wasser - bis 10 ml.

Beispiel 2

Salbe: Bestandteile in g für 18 g Salbe:
Nigerizin - 0,18; Ethanol 95% - 1,0; Vaseline - 15,987; Nipagin - 0,03; Nipasol - 0,003.

Beispiel 3

Suppositorien: Bestandteile in g pro Suppositorium von 3 g:
Nigerizin - 0,003; Aerosil 300 - 0,06; Vitepsol H 15 - 2,63; Miglinol 812 - 0,3.

Beispiel 4

Ergebnisse der toxikologischen und pharmakologischen Untersuchungen des erfindungsgemäßen Mittels (Nigerizin):
Bei örtlicher Anwendung zeigt Nigerizin keine toxische Wirkung am Auge eines Kaninchens bei Dosen, die 20 mal die therapeutischen Konzentrationen überschreiten, selbst bei lang andauernder Behandlung von 10 Tagen. Eine Salbe mit einer Konzentration von 2 % verursacht keine Erscheinungen von Reizen und Beschädigung auf der denudierten Haut von Kaninchen bei einer 20tägigen Behandlung. Eine 2%ige Salbe hat keine sensibilisierende Wirkung auf Meerschweinchen, auch nach lang andauernder Behandlung. Eine 2%ige Salbe übt keine embryotoxische und terratogene Wirkung auf weiße trächtige Ratten aus.

Bei Spritzenbehandlung:
Die akute Toxizität bei weißen Ratten und Mäusen (= $LD_{50}$) liegt im Bereich von 55 bis 66 mg/kg. Die chronische sechsmonatige Toxizität in Dosen bis 1/10 der $LD_{50}$ beträgt bis zu 5,6 mg/kg. Nigerizin weist keine Abweichungen der behandelten Ratten hinsichtlich folgender Kenndaten auf: Blutdiagramm, Blutgerinnung, biochemische Kennziffern des Blutes und des Harns, Leberproben, histologische Untersuchungen aller Organe, allgemeiner Zustand, Gewicht und Verhalten der Tiere. In Dosen bis zu 5,6 mg/kg ist keine embryotoxische und terratogene Wirkung bei der Untersuchung von trächtigen weißen Ratten beobachtet worden. Die pharmakologische Untersuchung zeigte gute Toleranz bei verschiedenen Tieren bei Konzentrationen bis zu 1/10 der $LD_{50}$. Nigerizin ruft keine Veränderungen im zentralen Nervensystem hervor, übt keine Wirkung auf das Herz-Blutgefäß-System von Katzen (gemessen an Blutdruck und Elektrokardiogrammen) aus, ändert nicht die Funktionen der Glattmuskulatur des Magen-Darm-Wegs bei Ratten und Kaninchen und zeigt keine Mutagenität.

Beispiel 5

Ergebnisse der klinischen Untersuchungen von Nigerizin (s. Tabelle 1).

Das Nigerizin enthaltende Arzneimittel in Form von Tropfen, Salbe und Suppositorien wurde an insgesamt 711 Patienten mit verschiedenen Virus-Erkrankungen: Labial-, Fazial-, Genital- und Gluteal- oder Femoralherpes, Herpes und Adenovirus-Keratitis und Konjunktivitis, Vaginitis, Endocervicitis und Exocervicitis mit Herpes-Ethiologie, Herpes Zoster der Augen und Haut angewendet.

TABELLE 1

Klinische Untersuchung von verschiedenen Verfahren zur Behandlung der Virus-Erkrankungen des Auges

| Verfahren (Mittel) | Klinische Form der Erkrankung | Zahl der Patienten | Prozent der günstigen Beeinflußung | Dauer der Behandlung (Tage) | Durchschnittsaufenthalt im Krankenhaus (Tage) |
|---|---|---|---|---|---|
| Nigerizin-Tropfen | Oberflächen-Herpeskeratitis | 66 | 87,7 | 8 | 9,4 |
| | Tiefe/Ulzerose/Keratitis | 19 | 76 | 15 | |
| | Adenovirus-Keratokonjunktivitis | 23 | 91,6 | 3 | |
| | Herpes Zoster-Kerato-Konjunktivitis | 4 | 100 | 14 | |
| Idoxyridin | Oberflächen-Herpes-Keratitis | 86 | 82 | 17,9 | 23,6 |
| | Tiefe/Ulzerose/Keratitis | 13 | 51 | 25,2 | |
| | Adenovirus Kerato-Konjunktivitis | 18 | 41 | 9,3 | |

Fortsetzung der Tabelle 1

| | | | | | |
|---|---|---|---|---|---|
| Immuno-mittel | Oberflächen Herpes Keratitis | 11 | 75 | 18,6 | 26,3 |
| | Tiefe/Ulzerose/Keratitis | 8 | 43 | 26 | |
| Physiko-chemische Mittel | Oberflächen Herpes Keratitis | 30 | 72 | 19,9 | 26,8 |
| | Tiefe/Ulzerose/Keratitis | 12 | 41 | 27,5 | |

Nigerizin-Tropfen wurden an 112 Patienten in drei Augenkliniken des Medizinischen Akademie-Forschungsinstitutes für Augenkrankheiten, Lehrstuhl für Ophthalmologie an den Hochschulen für Medizin in Varna und in Pleven verabreicht. Die Ergebnisse zeigen sehr gute Heileigenschaften des Nigerizins, welche die des Präparats Idoxyridin übertreffen sowie der bis jetzt verwendeten immunen Mittel (GammaGlobulin und Autohämotherapie und physiko-chemischen Mittel (Abrasio, Touchieren mit Iodtinktur usw.). Der Vorteil

des Nigerizins ist besonders deutlich hinsichtlich der Dauer der Behandlung, die bei Nigerizin um mehr als zweimal verringert wird, entsprechend dem durch Untersuchungen festgestellten durchschnittlichen Aufenthalt in den Krankenhäusern. Noch wichtiger ist das Vermeiden des Risikos der Beschädigung der Hornhaut und der Sehfähigkeit der Patienten.

Nigerizin in Form von Salbe wurde an 506 Patienten mit verschiedenen Herpes-Erkrankungen erprobt (siehe Tabelle 2).

TABELLE 2

| Heilwirkung von Nigerizin-Salbe bei Herpes-Erkrankungen | | | | | |
|---|---|---|---|---|---|
| Klinische Formen der Erkrankung | Zahl der Patienten | Kranke behandelt mit Nigerizin-Salbe | | Kontrollgruppe | |
| | | Patientenzahl | Prozent der Wirksamkeit | Patientenzahl | Prozent der Wirksamkeit |
| Labial-Herpes | 164 | 128 | 88,34 | 36 | 22 |
| Haut-Herpes | 110 | 87 | 87,77 | 23 | 18,9 |
| Genital-Herpes | 293 | 225 | 86,00 | 68 | 19,1 |
| Herpes-Zoster | 87 | 66 | 91,21 | 21 | 12,6 |
| Insgesamt | 654 | 506 | 87,58 | 148 | 18,85 |

Die Ergebnisse zeigen, daß die Daten der Untersuchungen der Nigerizin-Salbe um ein vielfaches besser sind als die der Kontrollgruppe, die mit bekannten Arzneimitteln behandelt wurde, sowie der Daten mit einer Placebo-Salbe. In drei Haut-Neurologischen Kliniken: Forschungsinstitut für Dermatologie und Geschlechtskrankheiten in Sofia und Lehrstühle für Dermatologie in den Medizinischen Hochschulen in Varna und Pleven wurde auch eine streng kontrollierte "Blinduntersuchung" (doppelter Blindversuch) durchgeführt, womit der spezifische Charakter der Beeinflussung der Herpes-Erkrankungen durch Nigerizin kategorisch bewiesen wurde (siehe Tabelle 3).

TABELLE 3

| Ergebnisse des "Doppelten Blindversuches" mit Nigerizin-Salbe | | | | | | | | |
|---|---|---|---|---|---|---|---|---|
| Klinische Formen der Erkrankung | Patientenzahl | Prozent der Beeinflußung mit Nigerizin | | | Patientenzahl | Prozent der Beeinflußung mit Placebo-Salbe | | |
| | | sehr gut | gut | nicht befriedigend | | sehr gut | gut | nicht befriedigend |
| Labial und Fazial-Herpes | 45 | 64,45 | 26,65 | 9,90 | 34 | 14,44 | 11,76 | 73,80 |
| Genital und Gluteal-Herpes | 95 | 62,10 | 25,26 | 12,64 | 74 | 9,59 | 8,10 | 82,30 |

Es wurde die Abhängigkeit zwischen der Wirksamkeit der Behandlung mit Nigerizin-Salbe und dem Beginn der Behandlung untersucht (siehe Tabelle 4).

TABELLE 4

| Abhängigkeit zwischen der Wirksamkeit der Behandlung mit Nigerizin-Salbe und dem Beginn der Behandlung | | | | | | |
|---|---|---|---|---|---|---|
| Behandlung nach Auftritt der Symptome | Prozent der Beeinflußung mit Nigerizin-Salbe | | | Prozent der Beeinflußung mit Placebo-Salbe | | |
| | Patientenzahl | Günstige Beeinflussung | Ohne Effekt | Patientenzahl | Günstige Beeinflussung | Ohne Effekt |
| I Tag | 40 | 100 | 0 | 28 | 17,85 | 82,15 |
| II Tag | 26 | 88,46 | 11,54 | 20 | 20 | 80 |
| III Tag | 22 | 54,54 | 45,46 | 10 | 30 | 70 |
| Insgesamt | 88 | 88,63 | 11,37 | 58 | 20,7 | 79,3 |

Bei 140 mit Nigerizin-Salbe behandelten Patienten wurde eine Wirksamkeit von 90% erreicht, verglichen mit weniger als 20% für die mit Placebo behandelten Kontrollgruppe. Die eingehende Analyse der Ergebnisse an 88 Patienten zeigt, daß es sehr wichtig ist, bei Nigerizin früh mit der Behandlung zu beginnen, wobei am ersten Tag die Wirksamkeit bis zu 100% beträgt. Bei den Patienten, die mit einer Placebo-Salbe behandelt wurden, wird die Wirksamkeit nicht durch die Phase der Erkrankung beeinflußt, was ihren nicht spezifischen Charakter beweist.

Nigerizin in Kapseln wurde im Forschungsinstitut für Gynäkologie in Sofia, sowie an den Lehrstühlen für Gynäkologie der Hochschulen für Medizin in Varna und in Pleven an 93 Frauen mit verschiedenen Formen von Herpes-Erkrankungen der Genitalien überprüft. Die Daten der klinischen Untersuchungen zeigen die wesentlichen Vorteile der spezifischen Behandlung, was eine Wirksamkeit von 89% gegenüber 20% Beeinflussung der Erkrankung in der Kontrollgruppe von 66 Frauen aufweist, wobei die Erkrankung im Durchschnitt nach 18,6 Tagen vorüber war (siehe Tabelle 5).

TABELLE 5

| Heileffekt von Nigerizin-Kapseln bei verschiedenen Herpes-Erkrankungen | | | | | | | |
|---|---|---|---|---|---|---|---|
| Klinische Formen der Erkrankung | Prozent der Beeinflußung durch Nigerizin-Kapseln | | | | Prozent der Beeinflußung durch Placebo-Kapseln | | |
| | Patientenzahl | sehr gut | gut | nicht befriedigend | Patientenzahl | beeinflußt | nicht beeinflußt |
| EXOCERVICITIS HERPETYCA | 51 | 64,8 | 24,4 | 10,8 | 38 | 21 | 79 |
| VAGINITIS HERPETYCA | 23 | 69,2 | 21,6 | 9,2 | 13 | 19,5 | 80,5 |
| ENDOCERVICITIS HERPETYCA | 19 | 56,3 | 29,4 | 14,3 | 15 | 19,8 | 80,2 |

## Patentansprüche

1. Verwendung eines durch Streptomyces hygroscopicus produzierten Antibiotikums mit folgender Formel:

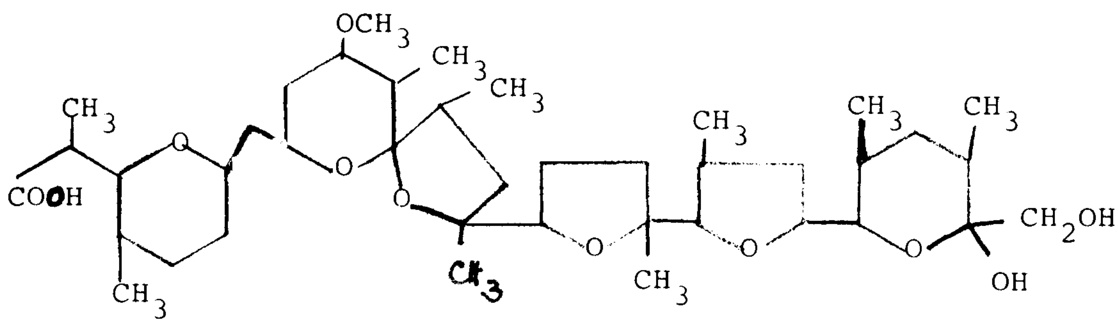

als Wirkstoff bei der Herstellung eines therapeutischen Mittels zur Behandlung von Viruserkrankungen, die durch DNS enthaltende Viren verursacht werden.

2. Verwendung des Antibiotikums nach Anspruch 1, wobei der Wirkstoff in Mengen von 0,001 bis 3,0 Gewichtsteilen, bezogen auf die Gesamtmenge des therapeutischen Mittels, vorliegt.

## Claims

1. Use of an antibiotic, produced by Streptomyces hygroscopicus, of the formula

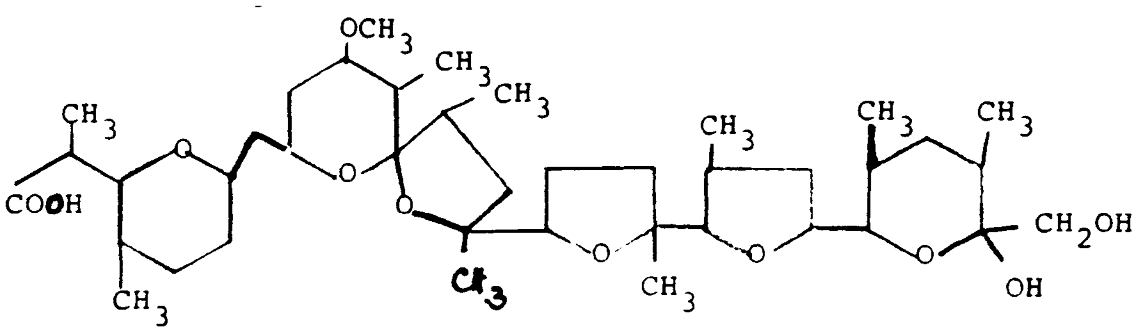

as active compound in the production of a therapeutic agent for treating viral diseases caused by DNA containing viruses.

2. Use of an antibiotic according to Claim 1 in which the amount of the active compound is from 0.001 to 3.0 parts by weight, calculated on the total amount of the therapeutic agent.

## Revendications

1. Utilisation d'un antibiotique, produit par le Streptomyces hygroscopicus, de formule:

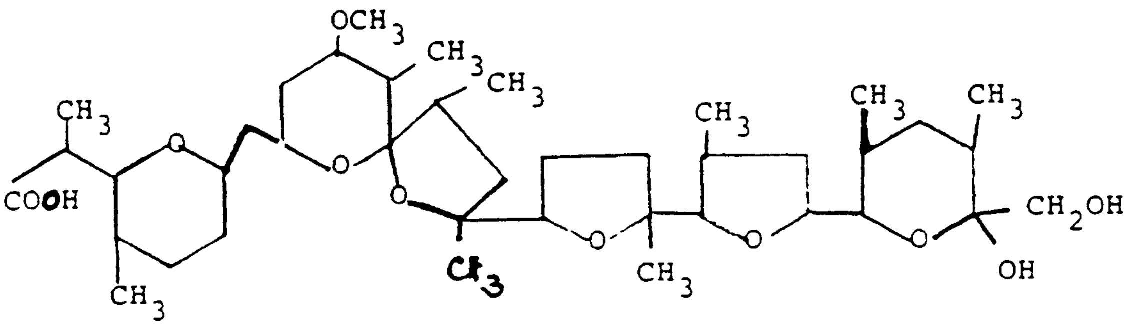

en tant que principe actif pour la fabrication d'un médicament par le traitement de maladies virales provoquées par des virus contenant de l'ADN.

2. Utilisation de l'antibiotique selon la revendication 1, dans laquelle le principe actif représente de 0,001 à 3,0 parties en poids du poids total du médicament.